(19) Europäisches Patentamt
European Patent Office
Office européen des brevets

(11) EP 1 962 817 B1

(12) EUROPEAN PATENT SPECIFICATION

(45) Date of publication and mention of the grant of the patent:
**17.11.2010 Bulletin 2010/46**

(21) Application number: **06848981.4**

(22) Date of filing: **21.12.2006**

(51) Int Cl.:
***A61K 9/70*** (2006.01) ***A61K 31/55*** (2006.01)
***A61K 9/00*** (2006.01)

(86) International application number:
**PCT/US2006/048783**

(87) International publication number:
**WO 2007/075883 (05.07.2007 Gazette 2007/27)**

(54) **TRANSDERMAL DELIVERY OF A SALT FORM OF MEPTAZINOL**

TRANSDERMALE VERABREICHUNG EINES MEPTAZINOLSALZES

ADMINISTRATION TRANSDERMIQUE D'UN SEL DE MEPTAZINOL

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.12.2005 US 753357 P**
**19.10.2006 US 862114 P**

(43) Date of publication of application:
**03.09.2008 Bulletin 2008/36**

(73) Proprietor: **Shire Pharmaceuticals, Inc.**
**Wayne, PA 19087-8301 (US)**

(72) Inventor: **FRANKLIN, Richard**
**Hampshire GU51 4NQ (GB)**

(74) Representative: **Goddar, Heinz J. et al**
**Forrester & Boehmert**
**Pettenkoferstrasse 20-22**
**80336 München (DE)**

(56) References cited:
**WO-A-2004/017941**
**WO-A-2004/098567**
**GB-A- 1 539 277**
**GB-A- 2 122 895**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 962 817 B1

## Description

### FIELD OF THE INVENTION

This invention relates to the administration of a salt of meptazinol for analgesic purposes and more particularly to a method and device for administering salt of meptazinol to a patient in need thereof over an extended period of time at an essentially constant rate while avoiding first pass metabolism.

### BACKGROUND OF THE INVENTION

Inadequate pain relief continues to represent a major problem for both patients and healthcare professionals. Optimal pharmacologic management of pain requires selection of the appropriate analgesic drug that achieves rapid efficacy with minimal side effects.

Mild analgesics are readily available and over the counter (OTC) analgesics such as paracetamol, are well established in mild pain. Stronger analgesics, as well as often requiring regular medication (3-4-times per day, minimum) have significant side effects e.g. gastric haemorrhage and/or ulceration with NSAIDs; constipation is a significant side effect of the milder opiates e.g. codeine and dihydrocodeine, while the stronger, prescription opiate analgesics, e.g. tramadol, effect cognition and self-awareness, in addition to gastrointestinal side effects.

Current therapy for the management of moderate to severe pain is sub-optimal. The strongest analgesics e.g. pethidine, fentanyl, morphine and diamorphine whilst being adequate analgesics, also have significant well known side effects that often limit use e.g. tolerance over time, gastrointestinal side effects and respiratory depression. In addition, use of the strongest analgesics is strictly controlled because of their addictive properties.

Meptazinol is a mixed agonist-antagonist analgesic with specificity for the mul opioid receptor which displays both opioid and cholinergic properties and its chemical structure is defined by formula (I) below:

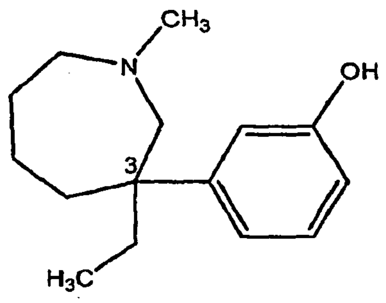

(I)

Preparation of meptazinol hydrobromide salt was referred to in U.S. Patent 3,729,465 and preparation of the free base form of meptazinol was referred to in U.S. Patent 4,197,241.

Meptazinol's cholinergic properties are thought to contribute to its anti-nociceptive effects and to minimize the usual range of opioid side effects. Meptazinol has been shown to have a negligible clinical dependency liability from both formal clinical investigation and the lack of reported instances of street use/abuse. The lack of addictive potential for meptazinol was first reported in 1987 by the internationally renowned investigator, Dr. Don Jasinski (Lexington, Kentucky). This property distinguishes meptazinol from many other strong analgesics such as fentanyl (e.g. Duragesic), pentazocine, oxycodone (e.g. Oxycontin, Percocet), and morphine which are all classified as "Controlled Drugs" with consequent prescription/ dispensation restrictions.

Meptazinol also has many clinical advantages over the more conventional opioid analgesics which include causing minimal respiratory depression, causing minimal sedation and lacking a constipating effect.

Causing minimal respiratory depression makes meptazinol a favored obstetric analgesic to avoid infant respiratory distress. Other analgesics given during labor such as pethidine and diacetylmorphine can cause significant infant respiratory depression giving rise to the so-called grey baby syndrome often necessitating the use of a narcotic antagonist such as naloxone to reverse this effect.

Causing minimal sedation is advantageous in treating chronic pain conditions and assists a patient in conducting a normal daily life. The sedation associated with other analgesics frequently induces lethargy and a dramatic reduction in the quality of life - with the patients entering a near twilight world.

Lacking a constipating effect is an important property in treating chronic pain. The constipation commonly associated with the other strong analgesics can be a most distressing condition especially for the older patient. For this

group of patients, frequently the target population for strong analgesics, the lack of a constipating effect for meptazinol represents an important advantage over other strong analgesics such as pethidine.

Additionally, age is unlikely to affect the clearance of meptazinol which is effected by a simple one-step glucuronidation process with the ensuing inactive, water-soluble conjugate being filtered at the kidney. This process of conjugative metabolic clearance is not as affected by age as some other clearance mechanisms such as direct filtration of the active entity at the kidney or oxidative metabolic clearance as required for example by pethidine.

However, despite these clinical advantages, use of meptazinol has been restricted by two major disadvantages: (1) low oral bioavailability; with reported mean values lying between 4-9% as the result of extensive first pass metabolism and (2) a propensity, in common with other strong analgesics, to cause nausea and emesis. The nausea and emesis worsens bioavailability due to physical drug loss by vomiting. Furthermore, since meptazinol is known to inhibit gastric emptying and effectively traps part of the orally dosed drug in the stomach, greater quantities of meptazinol may be lost through such emesis.

All these factors lead to highly variable plasma drug levels of meptazinol after oral dosing and consequently a variable patient response. Such is the demand for immediate relief from moderate to severe pain that a patient may be unwilling to continue treatment with meptazinol until an optimal dosage is discovered for their personal use. This frustration, in attaining optimal dosage levels for each individual patient, can lead to compliance problems and ineffective medication and pain relief. The compliance issue is further exacerbated by the need for frequent oral administration of meptazinol, typically 4-6 times per day as a result of its short plasma half-life (1.5-2.0 hours).

Examples of pharmaceutical compositions comprising meptazinol hydrochloride in combination with ibuprofen in the form of tablets, have been disclosed in GB 2 122 895.

Transdermal delivery of strong analgesics in recent years has proven to be a useful alternative to injectable delivery as a means of overcoming many of the problems associated with their oral administration. Modulating the sharp rise in plasma drug levels, usually seen after oral dosing, may serve to minimize the emesis associated with the comparatively high $C_{max}$ values resulting from rapid absorption. In the specific case of meptazinol, avoidance of emesis becomes more important to minimize loss of drug trapped in the stomach by its inhibitory effects on gastric emptying.

Transdermal delivery also provides a means of avoiding the first pass metabolism through the liver which in the case of meptazinol removes up to 98.1 % of an oral dose. Such a high first pass elimination of the drug inevitably leads to large inter and intra subject variability in achieved plasma drug concentrations. For example, in one publication (Norbury H.M, Franklin, R.A, Graham, D.F., Eur. J. Clin. Pharm., vol. 25, pgs 77-80, (1983)) oral bioavailablity varied from 1.89% to 18.5%, almost a ten-fold range.

Meptazinol is inherently not a potent drug when administered orally, requiring 200 mg dosages every four to six hours. Even when the poor bioavailability of meptazinol is factored in, the average daily required dose for an effective dose would be ~50 -100 mg which approximates to a flux rate of ~83-166 $\mu g/cm^2/h$ from a 25 $cm^2$ transdermal patch. Such inherently high flux rates are not usually seen with other transdermal products and so this represents a significant technical challenge.

Examples of transdermal delivery systems which generically refer to opioid analgesics including meptazinol have been referred to in the art, e.g. Oshlack et al. (U.S. Patent 6,716,449); Simon (U.S. Patent Application Publication 2004-0024006); Klose et al. (U.S. Patent Application Publication 2004-0028625); Cassell (U.S. Patent Application Publication 2006-0029654); Shevchuk et al. U.S. Patent Application Publication 2004-033253) and Schlagheck (U.S. Patent Application Publication 2006-240128).

However, none of these references recognized the problem high flux rate which is uniquely associated with meptazinol and were directed toward solving the problem of delivering other types of opioid drugs (Oschlack - morphine/hydromorphone, naltrexone, oxycodonelliydrocodone; Simon - nalmefene; Klose - fentanyl; Cassell - lidocaine; Shevchuk - naltrexone, fentanyl, oxycodone + acyl opioid antagonist; Schlagheck - opioid - N-methyl-D-aspartate antagonist). There is no evidence in that any of these references solved the problem of delivering meptazinol at the necessary high flux rates or any discussion as to how this problem could be solved.

Therefore, a need still exists in the art for a transdermal delivery system for a non-addictive mixed agonist-antagonist analgesic such as meptazinol to achieve a sufficiently high flux rate to deliver a pharmacologically effective amount of the drug to treat pain or provide analgesic relief.

Citation or identification of any document in this application is not an admission that such document is available as prior art to the present invention.

## SUMMARY OF THE INVENTION

Surprisingly, the applicants have found that some or all of the disadvantages in the art with respect to the use of meptazinol can be overcome by various delivery vehicles in a transdermal device, (and most unexpectedly) with use of particular salt forms of meptazinol to provide sufficiently high flux rates to achieve plasma concentrations effective for analgesic relief.

Thus, it is an object of this invention to provide a delivery system which avoids first pass metabolism and delivers a pharmacologically effective amount of meptazinol for pain or to provide analgesic relief. The invention provides a viable means of avoiding the very large first pass effect seen with meptazinol after oral dosing. This invention will result in lower variability in achieved plasma concentrations, improved analgesic efficacy and better patient compliance.

Patient compliance will be further improved by the requirement for less frequent dosage due to the sustained plasma concentrations achieved from this transdermal delivery device according to the present invention.

Additionally, the relatively slower rise in plasma drug concentrations is expected to minimize the drug's emetic effects which again will contribute to minimizing variability in analgesically effective plasma drug concentrations and improving patient compliance.

The terms "delivery system " and "delivery vehicle" as used herein is meant to describe a method of providing meptazinol via transdermal transportation which avoids "first pass metabolism". First pass metabolism refers to the reduction of bioavailability of a drug, e.g. meptazinol, because of the metabolic or excretory capacity of the liver which is a common problem associated with oral administration. Transdermal delivery is distinct from parenteral or delivery by injection in that the latter bypasses the stratum corneum, epidermal and dermal layers of the skin and delivers the active agent directly to the subcutaneous layer. Transdermal delivery as used herein is meant to describe a process wherein an active agent, e.g. a salt form of meptazinol, contacts and passes through or permeates through one or more of the stratum corneum, epidermal and dermal layers of the skin. This passing through or permeation through can be accomplished by means such as, but not limited to:

(1) transcellular penetration (across the cells);
(2) intercellular penetration (between the cells); or
(3) transappendageal penetration (via hair follicles, sweat and sebum glands, and pilosebaceous apparatus)
(4) by predisposing the stratum corneum to allow the passage of drug such as the use of thermal ablation technologies.
(5) exploiting natural transport mechanism in the skin such as that for phosphorylated vitamin E
(6) facilitated passage through the uppermost layers of the skin using microneedles

The invention disclosed herein is meant to encompass all pharmaceutically acceptable salts of meptazinol (including those of the weakly acidic phenolic function as well as those of the weakly basic azepine nitrogen). Furthermore, various other meptazinol precursors derived by covalent linkage to the phenolic function such as ethers esters and glycosides are described later. The pharmaceutically acceptable salts (of the phenol) include, but are not limited to, metal salts such as sodium salt, potassium salt, cesium salt and the like; alkaline earth metals such as calcium salt, magnesium salt and the like; organic amine salts such as triethylamine guanidine & *N*-substituted guanidine salts, acetamidine & *N*-substituted acetamidine salts, pyridine salt, picoline salt, ethanolamine salt, triethanolamine salt, dicyclohexylamine salt, N,N'-dibenzylethylenediamine salt and the like. Pharmaceutically acceptable salts (of the azepine) include, but are not limited to inorganic acid salts such as hydrochloride, hydrobromide, sulfate, phosphate and the like; organic acid salts such as trifluoroacetate, maleate, and the like; sulfonates such as methanesulfonate, ethanesulphonate, benzenesulfonate, p-toluenesulfonate, camphor sulphonate and naphthalenesulphonate, and the like; amino acid salts such as alaninate, asparginate, glutamate and the like.

Meptazinol is a chiral molecule containing one stereogenic center at the C-3 position of the azepine and can therefore exist as two enantiomeric forms (*R* and *S* stereoisomers).

Reference to meptazinol for the purposes of this invention encompasses each enantiomer and mixtures thereof including a racemic mixture (racemate) of the enantiomers unless otherwise indicated.

These and other embodiments are disclosed or are apparent from and encompassed by, the following Detailed Description.

## BRIEF DESCRIPTION OF THE DRAWINGS

The following detailed description, given by way of example, but not intended to limit the invention solely to the specific embodiments described, may best be understood in conjunction with the accompanying drawings, in which:

Figure 1 illustrates the comparative permeation of meptazinol free base and a number of its salts through human skin.
Figure 2 shows the skin flux of the various salts of meptazinol though human skin.
Figure 3 shows an example of a meptazinol containing transdermal patch
Figure 4 shows the plasma drug concentration time profile after repeated patch application to a minipig.

## DETAILED DESCRIPTION

The present invention is directed to a delivery system which delivers a pharmacologically effective amount of

meptazinol for pain or to provide analgesic relief. Examples of alternative delivery systems, include but are not limited to those means which enable delivery of meptazinol or salt form thereof via parenteral injection, pulmonary absorption, topical application, sublingual administration and rectal administration (e.g. suppositories). Parenteral injections include delivery via intravenous injection, subcutaneous injection, intramuscular injection, intraarterial injection and intrathecal injection. Pulmonary absorption includes the use of inhalants and aerosols. Topical administration includes administration via: (1) mucous membranes which includes but is not limited to mucous membranes of the conjunctiva, nasopharnyx, oropharynx, vagina, colon, urethra and urinary bladder; (2) the skin (which includes topical or transdermal delivery); and (3) the eye.

In one embodiment of the present invention, the delivery vehicle is for topical administration to the skin and includes a transdermal device which delivers a pharmacologically effective amount of meptazinol for pain or to provide analgesic relief.

The transdermal device is intended to deliver the pharmacologically effective amount of meptazinol either in a manner which: (1) controls the rate of drug delivery to the skin or (2) allows the skin to control the rate of drug absorption.

The transdermal device for the transdermal delivery of an effective amount of meptazinol to provide analgesic relief to a mammal or patient in need thereof comprises of:

(i) a backing layer;
(ii) a reservoir layer or compartment;
(iii) a controlling membrane or non controlling microporous membrane; and
(iv) an adhesive film which is optionally applied as a perimeter ring or as a geometric pattern or combination thereof;
(v) a release liner; and
wherein the reservoir layer or compartment contains a composition comprising

(a) a salt form of meptazinol in an amount which results in delivery of an effective amount of meptazinol when added into the device and said device is applied to the skin; and
(b) a pharmaceutically effective carrier.

An example of the perimeter ring or geometric pattern is shown in Figure 4, i.e. the adhesive film does not cover the entire surface area of the controlling membrane; the adhesive film is applied to enable contact with the skin while also allowing for the controlling membrane or non controlling microporous membrane to also contact the skin.

In one embodiment, the reservoir layer is a compartment and is formed from the control membrane or non controlling microporous membrane and the backing layer.

The backing layer, reservoir layer, control membrane, adhesive and release liner can be formed using conventional teachings in the art such as those referred to in U.S. Patent 6,818,226 (Dermal penetration enhancers and drug delivery systems involving same); U.S. 6,791,003 (Dual adhesive transdermal drug delivery system); U.S. Patent 6,787,149 (Topical application of opioid analgesic drugs such as morphine); U.S. Patent 6,716,449 (Controlled release compositions containing opioid agonist and antagonist); U.S. Patent 5,858,393 (Transdermal formulation); U.S. Patent 5,612,382 (Composition for percutaneous absorption of pharmaceutically active ingredients); U.S. Patent 5,464,387 (Transdermal delivery device); U.S. Patent 5,023,085 (Transdermal flux enhancers in combination with iontophoresis in topical administration of pharmaceuticals; U.S. Patent 4,891,377 (Trandermal delivery of the narcotic analgesics etorphine and analogs); U.S. Patent 4,654,209 (Preparation of percutaneous administration.

The transdermal device is able to provide long lasting relief and is an improvement from the prior art which require 4-6 dosages per day. In one embodiment the transdermal device is able to provide up to about 8 hours of analgesic relief; in another embodiment, the transdermal device is able to provide about 8 to about 24 hours of relief; and in a further embodiment, the Transdermal device is able to provide from about 24 hours of relief to about 168 hours of relief.

Another embodiment of the transdermal device may constitute a so-called "drug in adhesive" or matrix patch in which there is no reservoir layer but instead the drug is intimately distributed in an appropriate pressure sensitive adhesive such as but not limited to the DURO-TAK polyacrylates.

In yet another embodiment of the invention, the transdermal device comprises an array of microfabricated microneedles wherein the length of the microneedle(s) is long enough to penetrate the stratum corneum (outer 10-15 $\mu$m of the skin) and yet short enough so as not to stimulate the nerves deeper into the skin. Henry et al., "Microfabricated Microneedles: A Novel Approach to Transdermal Drug Delivery", J. Pharm. Sci., vol. 87: 922-925 (1998). The meptazinol containing composition is stored in the hollowed out section of the microneedle.

In a further embodiment the transdermal device consists of a disposable patch with an array of metallic filaments and a separate battery-operated electrical activator. A momentary pulse of current applied to the filaments through the activator creates numerous microchannels through the stratum corneum allowing the drug to subsequently permeate in a continuous manner.

A further embodiment exploits a natural transport mechanism in the skin to carry drugs across without disrupting the skin surface. This is based on the observation that phosphorylated vitamin E penetrates skin almost ten times faster than vitamin E itself. Microencapsulation of the drug within a shell of phosphorylated vitamin E, creating nanospheres, then enables the drug to be efficiently carried across the skin. Continuous delivery over an extended period is then achievable.

In another embodiment of the invention, transdermal drug delivery is enhanced by iontophoresis, magnetophoresis, or sonophoresis. Iontophoresis involves the delivery of charged chemical compounds across the skin membrane using an applied electrical field, *see e.g.* "Pharmaceutical Dosage Forms and Drug Delivery Systems - Chapter 10 - Transdermal Drug Delivery Systems, Ointments, Creams, Lotions and Other Preparations", ed. by Ansel et al., Williams & Wilkins, page 360, (1995). Magnetophoresis involves the use of a magnetic field to enhance drug delivery to the skin, *see e.g.* Murthy et al., "Physical and Chemical Permeation Enhancers in Transdermal Delivery of Terbutaline Sulphate", AAPS PharmSciTech. 2001; 2(1). Sonophoresis is the use of high-frequency ultrasound which serves to compromise the integrity of the stratum corneum layer and improve permeability of compounds through the skin.

Given the low solubility of the free base form of meptazinol free base (0.17 mg/mL in aqueous solution), it may be advantageous to derivatize the meptazinol to form a precursor compound (or a salt thereof) which will degrade into meptazinol when traversing the layer(s) of the skin. Transdermal delivery of meptazinol may be achieved by a transdermal device which contains a precursor of meptazinol which includes but is not limited to meptazinol esters, glycosides, salts of meptazinol or mixtures thereof. Precursors of meptazinol are compounds which undergo a transformation *in vivo* to produce meptanizol (e.g. cleavage of an ester bond, glycolysis, formation of the free base from the salt). Meptazinol esters, ethers and glycosides are compounds of the formula (II):

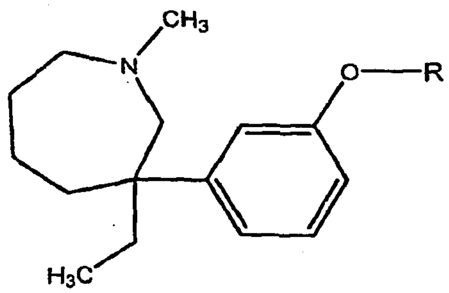

**(II)**

wherein R is an acyl group, a mono-, oligo- or poly-saccharide, or salts of mono-, oligo- or poly-saccharides. (Oligosaccharide indicates a saccharide comprised of 2-10 monosaccharide units which are covalently bonded together).

When R forms an ester, R is a -C(=O)-$C_1$-$C_{12}$-alkyl; or R is -C(=O)-$C_1$-$C_{12}$-alkyl-$NR_1R_2$ wherein $R_1$ and $C_{12}$-alkyl; or R is -C(=O)-$C_1$-$C_{12}$-alkyl-$NR_1R_2$ $R_2$ are independently hydrogen or $C_1$-$C_4$ alkyl; or R is C(=O)-$C_1$-$C_{12}$-alkyl$CO_2R_3$ wherein $R_3$ is hydrogen; $C_1$-$C_4$ alkyl or is a cation.

Alternatively, R is a -C(=O)-$C_1$-$C_4$-alkyl; or R is -C(=O)-$C_1$-$C_4$-alkyl-$NR_1R_2$ wherein $R_1$ and $R_2$ are independently hydrogen or $C_1$-$C_4$ allkyl; or R is C(=O)-$C_1$-$C_4$-alkyl$CO_2R_3$ wherein $R_3$ is hydrogen, $C_1$-$C_4$ alkyl or is a cation.

When R forms an ether, or R is a substituted or unsubstituted $C_1$-$C_{12}$-alkyl or substituted or unsubstituted aryl. Alternatively, R is a substituted or unsubstituted $C_1$-$C_4$-alkyl or substituted or unsubstituted phenyl. In both embodiments, the substituents are selected from the group consisted of halogen, $C_1$-$C_4$-alkyl, and $C_1$-$C_4$-alkoxy.

When R is a monosaccharide, R is selected from the group consisting of erythrosyl, threosyl, ribosyl, arabinosyl, xylosyl, lyxosyl, allosyl, altrosyl, glucosyl, glucosylamino, mannosyl, gulosyl, idosyl, galactosyl, galactosylamino, talosyl and salts thereof; or R is glusoyl, glucosylamino, galactosyl or galactosylamino and salts thereof; or R is glucosyl and salts thereof.

When R is an oligosacchande, R is selected from the group consisting of lactose, sucrose, trehalose, Lewis a trisaccharide, 3'-0-sulfonato Lewis a, Lewis b tetrasaccharide, Lewis x trisaccharide, Sialyl Lewis x, 3'-O-sulfonato Lewis x, Lewis y tetrasaccharide and salts thereof.

When R is a polysaccharide, R is selected from the group consisting of chitin, chitosan, cyclodextrin, dextran and pullulan; or the cyclodextrin is α-, β- or γ-cyclodextrin; or the cyclodextrin is β-cyclodextrin, dimethyl-β-cyclodextrin or hydroxypropyl-β-cyclodextrin.

As the cyclodextrin have a cavity which can accommodate the inclusion of a compound such as meptazinol, the cyclodextrins described in R above can also be added to meptazinol to form an inclusion complex rather than being linked covalently.

Alternatively, the meptazinol precusor is a salt and R is hydrogen but absent, whereby the oxygen is negatively charged; where the salt form is selected from the group consisting of sodium, potassium, caesium , calcium, magnesium, guanidine & N-substituted guanidine salts and acetamidine & N-substituted acetamidine salts triethylamine, pyridine,

picoline, ethanolamine, triethanolamine, dicyclohexylamine, N,N'-dibenzylethylenediamine. Alternatively, R is hydrogen - or one of the aforementioned substituents - and the azepine nitrogen is positively charged and linked with hydrochloride, hydrobromide, sulfate, phosphate, formate, acetate, trifluoroacetate, maleate, tartrate, methanesulfonate, ethanesulphonate, benzenesulfonate, p-toluenesulfonate, naphthalene sulphonate, camphor sulfonate, arginate, alaninate, asparginate, glutamate and mixtures thereof.

Surprisingly, and contrary to prior notions that lower melting points (mp) are normally associated with improved skin permeability, the azepine salts of meptazinol do not show such a relationship. For example, meptazinol hydrochloride (mp 184°C) was a much better permeant than the maleate (mp 102-104°C). The hydrochloride also displayed a higher flux rate than the camsylate (mp of 46-48°C).

Furthermore, and again in contrast to prior notions in the art, additional unexpected results occurred when using salts of meptazinol for transdermal delivery. Usually the free base is the preferred form of a drug for transdermal delivery due to its greater lipophilicty. For example, the skin flux of fentanyl free base is up to five times faster than the salt form. However, for meptazinol, the free base shows unexpectedly poor flux in comparison to the various salt forms. For example, meptazinol hydrochloride has a substantially greater flux than the free base. Previous reports in the scientific literature have suggested that ion pairs, i.e. salts, may improve transdermal flux by virtue of beneficially enhancing the physicochemical characteristics of the molecule. Such strategies have often employed lipophilic counter ions. Surprisingly, in the case of meptazinol, the use of more lipophilic counter ions such as the camsylate and tosylate were less effective in improving flux than the use of salts of stronger acid such as trifluoroacetic acid or hydrochloric acid.

A further factor improving the overall rate of skin flux was the unexpected radial or lateral diffusion of the meptazinol; this is advantageous in that higher skin fluxes can allow for smaller diameters of patch sizes

An additional analgesic can be added to the transdermal device. Examples of analgesics include but are not limited to ethanol, non-steroidal anti-inflammatory drugs (NSAIDs) and other compounds with anagelsic properties such as but not limited to amitriptyline and carbamazepine.

In another embodiment of the invention, the only analgesic present in the composition in the reservoir layer is a salt of meptazinol.

In another embodiment, the pharmaceutically effective carrier includes but is not limited to a solvent such as alcohol, isopropylmyristate, glycerol monooleate or a diol such as propylene glycol, or the like. The delivery of the salt form of meptazinol is enhanced by the use of a permeation enhancer which may also be included in the pharmaceutically effective carrier. Suitable permeation enhancers include but are not limited to polyunsaturated fatty acids (PUFA) such as arachidonic acid, lauric acid, α-linolenic acid, linoleic acid and oleic acid; dimethylisosorbide; azones; cyclopentadecalactone; alkyl-2-(N, N-disubstituted amino)-alkanoate ester (NexAct); 2-(n-nonyl)-1,3-dioxaolane (SEPA); cod-liver oil; essential oils, glycerol monoethers derived from saturated fatty alcohols; D-limonene; menthol and menthol ethyl ether; N-methyl-2-pyrrolidone (NMP); phospholipids; squalene; terpenes; and alcohols such as methanol, ethanol, propanol and butanol. *see e.g.* Pharmaceutical Skin Penetration Enhancement, ed. Walters et al., Marcel Dekker, Inc., (1993); Williams et al., "Penetration Enhancers", Adv. Drugs Deliv. Rev., vol. 56, pgs 603-618, (2004).

Alternatively, in another embodiment of the invention transdermal drug delivery may be effected using various topically applied ointments, creams, gels or lotions. Typically these may comprise oil-in-water emulsions or water-in-oil emulsions incorporating the salt form of meptazinol in one of the preferred vehicles. Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

The transdermal drug delivery can be made concomitant with oral administration of a composition containing an analgesic agent.

In another embodiment of the invention, the solubility (as measured in aqueous solution) of the salt form of meptazinol is 30 mg/mL to 500 mg/mL; in yet another embodiment of the invention, the solubility is 50 mg/mL to 400 mg/mL; and in a still further embodiment of the invention, the solubility is 75 mg/mL to 300 mg/mL.

Skin flux can be determined by multiplying the permeability coefficient ($k_p$ in cm/h) of meptazinol by the aqueous solubility of meptazinol. The aqueous solubility of meptazinol (free base) is 0.17 mg/mL and the permeability coefficient of meptazinol can be calculated using the empirical formula:

$$\log k_p = -2.7 + 0.71 \log P - 0.0061\ \mathrm{MW} \quad (\mathrm{MW\ for\ meptazinol\ is\ 233.35})$$

This results in an estimated skin flux of just 5.6 μg/cm$^2$/h for meptazinol which is roughly 15-30 x lower than the flux rate necessary to achieve analgesic effects through transdermal delivery.

In another embodiment of the invention, the skin flux for the delivery of the salt form of meptazinol, is 20 to 1000 μg/cm$^2$/h; in yet another embodiment of the invention, the skin flux for the delivery of the meptazinol or meptazinol

precursor is 50 to 500 μg/cm$^2$/h; in a further embodiment of the invention is 125 to 250 μg/cm$^2$/h and in a still further embodiment of the invention is 160 to 200 μg/cm$^2$/h.

In another embodiment of the invention the pH of the environment into which the salt form of meptazinol is released is pH 2 .0 to pH 4.0; in another embodiment pH 4.0 to pH 7.0; in yet another embodiment, the pH is 4.0 to 6.0; and in a further embodiment, the pH is 4.0 to 5.0.

Optionally, additional skin care ingredients may be combined with the salt form of meptazinol for their art recognized effects, these include abrasives, absorbents, adhesives, antiacne agents, anticaking agents, anticareis agents, antidandruff agents, antifoaming agents, antifungal agents, antimicrobial agents, antioxidants, antiperspirant agents, antistatic agents, binders, buffering agents, bulking agents, chelating agents, colorants, corn/callus/wart removers, corrosion inhibitors, cosmetic astringents, cosmetic biocides, denaturants, depilating agents, drug astringents, emollients, emulsion stabilizers, depilating agents, exfoliants, external analgesics, film formers, flavoring agents, fragrance ingredients, gelling agents, humectants, lytic agents, occlusives opacifying agents, oxidizing agents, pesticides, pH adjusters, plasticizers, preservatives, propellants, reducing agents, skin-bleaching agents, skin-conditioning agents, skin protectants, slip modifiers, solvents, sunscreen agents, surface modifiers, surfactants (including cleansing agents, emulsifying agents, foam boosters, hydrotopes, solubilizing agents, suspending agents), suspending agents (non-surfactant), ultraviolet light absorbers, viscosity controlling agents, viscosity decreasing agents, viscosity increasing agents (aqueous), viscosity increasing agents (non-aqueous) and mixtures thereof.

These additional skin care ingredients include but are not limited to those described in The International Cosmetic Ingredient Dictionary and Handbook, 9th Edition (2002); Remington - The Science and Practice of Pharmacy, 21st Edition (2005), Goodman & Gilman's The Pharmacological Basis of Therapeutics, 11th Edition (2005) and Ansel's Parmaceutical Dosage Forms and Drug Delivery Systems (8th Edition), edited by Allen et al., Lippincott Williams & Wilkins, (2005).

Controlling the release rate of the meptazinol composition and avoiding compromising the stickiness of the perimeter ring of the adhesive layer of the transdermal device is also desired. As such another embodiment is and a gelling agent to the meptazinol composition. Suitable gelling agents include but are not limited to Klucel (hydroxypropyl cellulose) and Carbopol 980. Such gelling agents, by virtue of the viscosity they provide, will control and restrict the rate of vehicle delivery through the microporous membrane. Typically this may be of the order of 1.5-3.uL/cm$^2$/h. By such control of the rate of delivery of drug and vehicle to the skin surface this may serve to limit any unwanted skin irritancy.

One of the side effects of transdermal delivery of an active agent is indeed the occurrence of skin irritation. While not wishing to be bound by theory, meptazinol may cause skin irritation due to the formation of oxidized or degradative products (e.g. meptazinol 1,4 quinone, meptazinol dimers). Furthermore, there is a possibility of dimerization of this quinone could be responsible for the pronounced yellow discoloration of the gel observed on standing. It has been found that this discoloration can be completely eliminated by the inclusion of the antioxidant butylated hydroxy toluene (BHT - 0.02- 0.05%). Ascorbic acid (0.05%) also afforded some reduction in this yellow discoloration, but other anti-oxidants such as butylated hydroxy anisole, alpha tocopherol and pyrogallol did not. Lesser amounts of BHT and ascorbic acid may also be suitable for the meptazinol compositions of the invention.

As such, another embodiment is a meptazinol containing composition for transdermal delivery which is free from skin irritation which further comprises an antioxidant selected from the group consisting of BHT, ascorbic acid and mixtures thereof.

Another factor which could induce skin irritation could, in part, be an inappropriate pH especially lower pH's. This can be raised by the use of a pharmaceutically acceptable basifying agent such as, but not exclusively restricted to, diethanolamine, disopropanolamine or tromethamine(TRIS).

In another embodiment of the invention, use of the transdermal device described hereinabove can be used to provide analgesic effects to treat systemic or localized pain to a patient in need thereof.

Yet another embodiment is a method of delivering meptazinol which avoids first pass metabolism which comprises of transdermal delivery. In another embodiment of this invention the method of delivery is non-oral and/or non-parenteral.

Other advantages and characteristics of the invention will become apparent on reading the following description, given by way of non-limiting examples.

## Examples

Example 1 - Improved Skin Flux by Using Meptazinol HCl Salt

Using human skin in a conventional Franz cell in vitro apparatus the transdermal permeation of meptazinol was measured by assaying the amount of drug in the receptor fluid beneath the skin sample at various times after application to the skin. Figure 1 shows that a salt of meptazinol is surprisingly more permeable than the free base form of meptazinol. Figure 2 shows that surprisingly meptazinol salts formed from a stronger acid, such as the hydrochloride

and trifluoroaceate salt are more rapidly absorbed than are those of weaker organic acids such as the camsylate, tosylate or maleate.

The data presented in Table 1 below show that under the test conditions cited above, the mean flux for the various salts tested were suitable for producing concentrations of meptazinol sufficient to produce a long-lasting effect when administered to a patient in need thereof.

**Table 1: Intersubject variability in flux rates for meptazinol salts through human skin**

| Applied compound | Cell ID | Donor number | Flux ug/cm$^2$/h | Mean flux ± sd |
|---|---|---|---|---|
| M HCl (1) | 1 | 281 | 197.1 | |
| M HCl (1) | 2 | 282 | 194.0 | |
| M HCl (1) | 3 | 284 | 162.5 | 173.3±35.2 |
| M HCl (1) | 4 | 287 | 135.7 | |
| M HCl (1) | 5 | 289 | 218.0 | |
| M HCl (1) | 6 | 295 | 132.6 | |
| M camsylate | 7 | 281 | 78.1 | |
| M camsylate | 8 | 282 | 110.9 | |
| M camsylate | 9 | 284 | 27.3 | 79.2± 71.2 |
| M camsylate | 10 | 287 | 45.1 | |
| M camsylate | 11 | 289 | 204.2 | |
| M camsylate | 12 | 295 | 9.5 | |
| M tosylate | 13 | 281 | 158.8 | |
| M tosylate | 14 | 282 | 165.0 | |
| M tosylate | 15 | 284 | 90.9 | 135.9 ± 82.5 |
| M tosylate | 16 | 287 | 39.6 | |
| M tosylate | 17 | 289 | 273.7 | |
| M tosylate | 18 | 295 | 87.4 | |
| M HCl (2) | 19 | 292 | 66.5 | |
| M HCl (2) | 20 | 288 | 222.6 | 179.0 ±75.1 |
| M HCl (2) | 21 | 282 | 213.4 | |
| M HCl (2) | 22 | 281 | 213.4 | |
| M TFA | 23 | 292 | 29.1 | |
| M TFA | 24 | 288 | 422.2 | 224.4 ±167.6 |
| M TFA | 25 | 282 | 163.8 | |
| M TFA | 26 | 281 | 282.3 | |
| M maleate | 27 | 292 | 41.3 | |
| M maleate | 28 | 288 | 91.3 | 78.0 ± 24.7 |
| M maleate | 29 | 282 | 85.3 | |
| M maleate | 30 | 281 | 93.9 | |
| NB Vehicle comprised 2% oleic acid: 2% dimethyl isosorbide: 96% propylene glycol | | | | |

The data in Table 2 was obtained using the same in vitro Franz cell technique verifies that there was surprisingly no correlation between lower melting points and higher solubilities with overall skin flux rates. For example, based on prior notions in the art, meptazinol hydrochloride which has a higher melting point than meptazinol free base, would have been expected to have a worse skin flux rate but instead is several times better than the meptazinol free base. Likewise, meptazinol hydrochloride which is the salt of a stronger acid, has both lower solubility and higher melting point than meptazinol camsylate, tosylate or maleate which is the salt of a weaker acid, and yet still have an unexpectedly better

skin flux rate.

**Table 2: Saturated solubilities (& melting points) of Meptazinol free base and selected salts in a potential delivery vehicle comprising 2% oleic acid: 2% dimethyl isosorbide: 96% propylene glycol**

| Compound | Solubility at 32°C (mg/ml) | MP (°C) |
|---|---|---|
| Meptazinol hydrochloride | 78 | 184-186 |
| Meptazinol camsylate | ~250** | 46-48 |
| Meptazinol tosylate | ~140** | 40-42 |
| Meptazinol trifluoroacetate | 170* | 112-114 |
| Meptazinol maleate | 105* | 102-104 |
| Meptazinol free base | ~20** | 128-133 |
| * Measured by HPLC<br>**Estimated from visual assessment only | | |

Example 2 - Meptazinol Composition

Numerous studies using skin collected from cosmetic surgical procedures in women (usually 'tummy tucks') were conducted in order to establish and refine the composition of the transdermal gel. These studies culminated in the selection of a 3:2:95 weight ration of (OA:DI:PG) vehicle (OA - oleic acid; DI - dimethyl isosorbide; PG - propylene glycol)

A meptazinol gel composition for use with a transdermal patch was prepared by mixing together the following ingredients (all % by weight):

| | |
|---|---|
| 83.296% | Propylene glycol (PG) - EP (BASF and Inovene) |
| 2.63% | Oleic acid (OA) - Super Refined Oleic Acid NF/EP (Croda) |
| 1.754% | Dimethyl isosorbide (DI) - Arlasolve™ (Uniqema) |
| 0.8% | Hydroxypropyl cellulose - Klucel HF grade NF/EP (Hercules) |
| 0.02% | Butylated hydroxytoluene (BHT) - EP grade (Fluka) |
| 11.5% | Meptazinol HCl BP grade (Kern Pharma) |
| Note: the weight ratio of (OA:DI:PG) in isolation is 3:2:95. | |

Example 4 - Meptazinol Containing Transdermal Patch

Figure 4 shows an example of a meptazinol containing transdermal patch which was prepared in accordance with the invention.

ScotchPak 9742 fluoropolymer with a thickness of 4.6 mil and 98 mm diameter was used to form the release liner (1). DSM Solupor 10PO5A which has a 55 mm diameter with a 6 mm perimeter heat seal flange was used to form the microporous membrane (2). Amcor C FILM (Amcor Flexibles Inc.) with a 6 mil thickness, 55 mm diameter with a 6 mm perimeter heat seal flange (or alternatively, 54 mm diameter with a 5 mm heat seal flange) was used to form the backing film (3). Dow Corning BIO PSA 7-4302 adhesive mixed with 2.5% of Dow Corning 200 fluid (tack enhancer) was used to form the adhesive ring (4) which has a diameter of 98 mm with a 50 mm diameter center hole (coating weight of the adhesive ring (4) was 85 g/m$^2$).

The drug reservoir is formed by the combination of the microporous membrane (2) and the backing film (3) and has a fill volume of 100 μL/cm$^2$. The meptazinol composition contained in the drug reservoir was a 2.5 mL composition described in Example 3.

Example 5 - In vivo transdermal absorption studies in Minipigs

Two studies were conducted in Göttingen minipigs, involving daily application of the patch of Example 4 for up to 7 days. This patch comprised a gel reservoir (100μL/cm$^2$, nominal initial volume 2.5 mL) over a Solupor 10PO5A microporous membrane (surface area 25 cm$^2$) This was secured to the skin via a perimeter ring of adhesive and an adhesive overlay. On a separate occasion, the pigs were given an i.v. dose of meptazinol at ~1 mg/kg to enable bioavailability to be determined.

The results of this study showed that between 150-200 mg of drug left the patch over a 24-hour period. The pharmacokinetic profile showed a negligible lag time and time of maximum concentration ($t_{max}$) occurring within 8 hours.

Plasma levels were sustained at steady state, with a mean fluctuation index of only 2.3. The transdermal bioavailability was low, being of the order of 8% to 12%, possibly as the result of skin metabolism in the minipigs. However, no such extensive glucuronidation has been reported for other phenolic analgesics applied to human skin (Roy SD, Hou SY, Witham SL, Flynn GL, "Transdermal delivery of narcotic analgesics: comparative metabolism and permeability of human cadaver skin and hairless mouse skin", J Pharm Sci., vol. 83(12):1723-8 (1994)).
This surprising gain in the rate of skin flux was thought to be the result of rapid radial or lateral diffusion of the meptazinol salt when a gelling agent was added.

Example 6 - Protocol for In vivo Skin Flux Determination in Humans

The following protocol is to be practiced to determine the transdermal flux of a meptazinol gel formulation and to determine the systemic availability compared to an intravenous (IV) administration. In addition, the protocol also allows for an assessment of safety and local tolerance of meptazinol transdermal gel compared to an IV (intravenous) administration of meptazinol and a gel placebo.
**Treatment A:** Meptazinol gel and/or patch as described in Examples 3 and 4 above, will be applied and secured to the inside of the lower arm of ten volunteers and occluded for 24 hours plus a concurrent single dose of intravenous placebo (sterile 0.9% w/v sodium chloride) 0.5mL injection infused over 30 seconds (slow IV injection). The meptazinol gel will be administered to provide for a total topical dose of 129 mg meptazinol (free base). The concentration of meptazinol hydrochloride in gel will be 100 mg/mL; 0.1 mL/cm$^2$ gel will cover a skin surface area of 13 cm$^2$. This will be contained within a stoma ring of 4cm diam. beneath which a DSM 10PO5A microporous membrane will be placed. The ring will be occluded with a watch glass.
**Treatment B:** Matching gel placebo applied and secured to the inside of the lower arm and occluded for 24 hours plus a concurrent 50 mg single dose of IV meptazinol 0.5 mL injection infused over 30 seconds (slow IV injection).
All subjects will receive both treatments over two dose periods. Subjects will be randomized with respect to the sequence of treatments will be selected to provide for adequate assessment of safety, tolerability, and pharmacokinetics.
This protocol is a randomized, two way cross over design for a treatment sequence (AB or BA). Treatments will be administered on days 1 and 4 of the study. Gel (meptazinol formulation or placebo) will be applied to the skin and left for 24 hours. IV meptazinol or the matching IV placebo will be administered as a slow bolus infusion in the arm opposite to that receiving gel. On day 1 gel will be applied to the right arm and on day 4 gel will be applied to the left arm.
A series of plasma and urine PK samples will be collected and skin will be assessed for tolerance of gel during the inpatient period of the study.
This protocol will evaluate meptazinol (and possible metabolites) plasma concentration-time profiles and pharmacokinetic parameters to include $C_{max}$, $t_{max}$, AUC, $t_{1/2}$ and bioavailability of meptazinol administered by transdermal gel relative to intravenous administration for each subject; estimation of transdermal flux; amount of meptazinol (and possible metabolites) excreted in urine; local tolerance assessed by visual inspection of skin, erythema and oedema, as well as testing the individual for itchiness, burning or other discomfort at the site of gel application supported by digital photography of application site.

## Claims

1. A transdermal device for the transdermal delivery of an effective amount of meptazinol to provide analgesic relief to a mammal or patient in need thereof which comprises:

    (i) a backing layer;
    (ii) a reservoir layer or compartment;
    (iii) a controlling membrane or non controlling microporous membrane;
    (iv) an adhesive film which is optionally applied as a perimeter ring or as a geometric pattern or combination thereof;
    (v) a release liner; and
    wherein the reservoir layer contains a composition comprising

        (a) a salt form of meptazinol in an amount which results in delivery of an effective amount of meptazinol when added into the device and said device is applied to the skin; and
        (b) a pharmaceutically effective carrier.

2. The transdermal device of claim 1, wherein the device further comprises a control membrane and an adhesive and optionally a release liner.

3. The transdermal device of claim 1, wherein the salt form is hydrochloride, hydrobromide, sulfate, phosphate, trifluoroacetate, maleate, tartrate, methanesulfonate, ethanesulphonate, benzenesulfonate, p-toluenesulfonate, naphthalene sulphonate, camphor sulfonate, alaninate, asparginate, glutamate or mixtures thereof.

4. The transdermal device of claim 3, wherein the salt form is hydrochloride, camphor sulphonate, toluene sulfonate, trifluoroacetate, maleate or mixtures thereof.

5. The transdermal device of claim 4 , wherein the salt of meptazinol has a solubility selected from the group consisting of 30 mg/mL to 500 mg/mL; 50 mg/mL to 400 mg/mL; and 75 mg/mL to 300 mg/mL.

6. The transdermal device of claim 4 , wherein the salt of meptazinol has a skin flux selected from the group consisting of 20 to 1000 $\mu g/cm^2/h$; 50 to 500 $\mu g/cm^2/h$; 125 to 250 $\mu g/cm^2/h$ and 160 to 200 $\mu g/cm^2/h$.

7. The transdermal device of claim 4 , wherein the salt of meptazinol is released into an skin environment with a pH selected from the group consisting of pH 2 to pH 4.0; pH 4.0 to pH 7.0; pH 4.0 to pH 6.0; and pH 4.0 to pH5.0.

8. The transdermal device of claim 1, which comprises of a salt of meptazinol wherein the salt form is hydrochloride, trifluoroacetate or mixtures thereof, has a solubility of 75 mg/mL to 300 mg/mL and a skin flux of 75 to 250 $\mu g/cm^2/h$.

9. The transdermal device of claim 6, which additionally comprises abrasives, absorbents, adhesives, antiacne agents, anticaking agents, anticareis agents, antidandruff agents, antifoaming agents, antifungal agents, antimicrobial agents, antioxidants, antiperspirant agents, antistatic agents, binders, buffering agents, bulking agents, chelating agents, colorants, corn/callus/wart removers, corrosion inhibitors, cosmetic astringents, cosmetic biocides, denaturants, depilating agents, drug astringents, emollients, emulsion stabilizers, epilating agents, exfoliants, external analgesics, film formers, flavoring agents, fragrance ingredients, humectants, lytic agents, occlusives, opacifying agents, oxidizing agents, pesticides, pH adjusters, plasticizers, preservatives, propellants, reducing agents, skin-bleaching agents, skin-conditioning agents, skin protectants, slip modifiers, solvents, sunscreen agents, surface modifiers, surfactants including cleansing agents, emulsifying agents, foam boosters, hydrotopes, solubilizing agents, suspending agent, non-surfactant suspending agents ultraviolet light absorbers, viscosity controlling agents, viscosity decreasing agents, aqueous viscosity increasing agents, non-aqueous viscosity increasing agents and mixtures thereof.

10. The transdermal device of claim 8, which additionally comprises abrasives, absorbents, adhesives, antiacne agents, anticaking agents, anticareis agents, antidandruff agents, antifoaming agents, antifungal agents, antimicrobial agents, antioxidants, antiperspirant agents, antistatic agents, binders, buffering agents, bulking agents, chelating agents, colorants, corn/callus/wart removers, corrosion inhibitors, cosmetic astringents, cosmetic biocides, denaturants, depilating agents, drug astringents, emollients, emulsion stabilizers, epilating agents, exfoliants, external analgesics, film formers, flavoring agents, fragrance ingredients, humectants, lytic agents, occlusives, opacifying agents, oxidizing agents, pesticides, pH adjusters, plasticizers, preservatives, propellants, reducing agents, skin-bleaching agents, skin-conditioning agents, skin protectants, slip modifiers, solvents, sunscreen agents, surface modifiers, surfactants including cleansing agents, emulsifying agents, foam boosters, hydrotopes, solubilizing agents, suspending agents, non-surfactant suspending agents, ultraviolet light absorbers, viscosity controlling agents, viscosity decreasing agents, aqueous viscosity increasing agents, non-aqueous viscosity increasing agent and mixtures thereof.

11. Use of the transdermal device of claim 1 in the manufacture of a medical device for the providing of an analgesic effect to a patient in need thereof.

12. The use of claim 11, wherein the analgesic effect is localized.

13. The use of claim 11, wherein the analgesic effect is systemic.

14. The use of claim 11, wherein administration of the transdermal device is accompanied by iontophoresis.

15. The use of claim 11, wherein administration of the transdermal device is accompanied by magnetophoresis.

16. The use of claim 11, wherein administration of the transdermal device is accompanied by sonophoresis.

**17.** The use of claim 11, wherein transdermal delivery is effected by use of a lotion, cream or ointment in place of a device.

**18.** The use of claim 11, wherein transdermal delivery is effected using a matrix patch in which the drug is dissolved in a suitable pressure sensitive adhesive.

**19.** The use of claim 11, wherein administration of the transdermal device is accompanied by thermal ablation.

**20.** The use of claim 11, wherein administration of the transdermal device is accompanied by a natural skin transporter of phosphorylated Vitamin E.

**21.** The use of claim 11, wherein administration of the transdermal device is accompanied by use of microneedles.

**Patentansprüche**

**1.** Transdermale Vorrichtung für die transdermale Verabreichung einer wirksamen Menge an Meptazinol, um schmerzlindernde Abhilfe für ein bedürftiges Säugetier oder einen Patienten bereitzustellen, welche umfasst:

(i) eine Verstärkungsschicht;
(ii) eine Reservoirschicht oder eine Kammer;
(iii) eine Steuerungsmembran oder eine nicht steuernde mikroporöse Membran;
(iv) einen Haftfilm, der optional als ein Perimeterring oder als ein geometrisches Muster oder eine Kombination derselben aufgetragen ist;
(v) einen Abziehdeckbogen; und

wobei die Reservoirschicht eine Zusammensetzung enthält, die umfasst:

(a) eine Salzform von Meptazinol in einer Menge, die in einer Verabreichung einer wirksamen Menge an Meptazinol resultiert, wenn sie in die Vorrichtung gegeben wird und die Vorrichtung auf die Haut appliziert wird; und
(b) einen pharmazeutisch wirksamen Träger.

**2.** Transdermale Vorrichtung nach Anspruch 1, wobei die Vorrichtung ferner eine Steuermembran und einen Haftstoff und optional einen Abziehdeckbogen umfasst.

**3.** Transdermale Vorrichtung nach Anspruch 1, wobei die Salzform Hydrochlorid, Hydrobromid, Sulfat, Phosphat, Trifluoracetat, Maleat, Tartrat, Methansulfonat, Ethansulfonat, Benzolsulfonat, p-Toluolsulfonat, Naphthalinsulfonat, Kampfersulfonat, Alaninat, Asparaginat, Glutamat oder Mischungen derselben ist.

**4.** Transdermale Vorrichtung nach Anspruch 3, wobei die Salzform Hydrochlorid, Kampfersulfonat, Toluolsulfonat, Trifluoracetat, Maleat oder Mischungen derselben ist.

**5.** Transdermale Vorrichtung nach Anspruch 4, wobei das Salz von Meptazinol eine Löslichkeit aufweist, die ausgewählt ist aus der Gruppe bestehend aus 30 mg/ml bis 500 mg/ml; 50 mg/ml bis 400 mg/ml; und 75 mg/ml bis 300 mg/ml.

**6.** Transdermale Vorrichtung nach Anspruch 4, wobei das Salz von Meptazinol einen Hautfluss aufweist, der ausgewählt ist aus der Gruppe bestehend aus 20 bis 1000 $\mu g/cm^2/h$; 50 bis 500 $\mu g/cm^2/h$; 125 bis 250 $\mu g/cm^2/h$ und 160 bis 200 $\mu g/cm^2/h$.

**7.** Transdermale Vorrichtung nach Anspruch 4, wobei das Salz von Meptazinol in eine Hautumgebung mit einem pH-Wert freigegeben wird, der ausgewählt ist aus der Gruppe bestehend aus pH 2 bis pH 4,0; pH 4,0 bis pH 7,0; pH 4,0 bis pH 6,0; und pH 4,0 bis pH 5,0.

**8.** Transdermale Vorrichtung nach Anspruch 1, welche ein Salz von Meptazinol umfasst, wobei die Salzform Hydrochlorid, Trifluoracetat oder Mischungen derselben ist, welches eine Löslichkeit von 75 mg/ml bis 300 mg/ml und einen Hautfluss von 75 bis 250 $\mu g/cm^2/h$ aufweist.

**9.** Transdermale Vorrichtung nach Anspruch 6, welche zusätzlich Poliermittel, Absorptionsmittel, Haftmittel, Antiaknemittel, Antibackmittel, Antikareismittel, Antischuppenmittel, Antischäummittel, Antipilzmittel, antimikrobielle

Mittel, Antioxidantien, Antischwitzmittel, antistatische Mittel, Bindemittel, Puffermittel, Auflockerungsmittel, chelatbildende Mittel, Farbstoffe, Hühnerauge-/Hornhaut-/Warzen-Entferner, Korrosionsinhibitoren, kosmetische Adstringentien, kosmetische Biozide, Denaturierungsmittel, Depilierungsmittel, Arzneimitteladstringentien, Weichmacher, Emulsionsstabilisatoren, Epilierungsmittel, Abschälmittel, externe Analgetika, Filmbildner, Aromastoffe, Duftinhaltsstoffe, Feuchthaltemittel, lytische Agentien, Okklusivmittel, opazifizierende Mittel, oxidierende Mittel, Pestizide, pH-Einstellungsmittel, Plastifizierungsmittel, Konservierungsmittel, Treibmittel, Reduktionsmittel, Hautbleichungsmittel, Hautkonditionierungsmittel, Hautschutzmittel, Abgleitmodifizierungsmittel, Lösungsmittel, Sonnenschutzmittel, Oberflächenmodifizierer, oberflächenaktive Mittel einschließend Reinigungsmittel, emulgierende Mittel, Schaumbooster, Hydrotope, löslich machende Mittel, nichtoberflächenaktive Suspensionsmittel, Absorber für ultraviolettes Licht, Viskositätssteuerungsmittel, Viskositätsabsenkungsmittel, wässrige Viskositätserhöhungsmittel, nicht wässrige Viskositätserhöhungsmittel und Mischungen derselben umfasst.

**10.** Transdermale Vorrichtung nach Anspruch 8, welche zusätzlich Poliermittel, Absorptionsmittel, Haftmittel, Antiaknemittel, Antibackmittel, Antikareismittel, Antischuppenmittel, Antischäummittel, Antipilzmittel, antimikrobielle Mittel, Antioxidantien, Antischwitzmittel, antistatische Mittel, Bindemittel, Puffermittel, Auflockerungsmittel, chelatbildende Mittel, Farbstoffe, Hühnerauge-/Hornhaut-/Warzen-Entferner, Korrosionsinhibitoren, kosmetische Adstringentien, kosmetische Biozide, Denaturierungsmittel, Depilierungsmittel, Arzneimitteladstringentien, Weichmacher, Emulsionsstabilisatoren, Epilierungsmittel, Abschälmittel, externe Analgetika, Filmbildner, Aromastoffe, Duftinhaltsstoffe, Feuchthaltemittel, lytische Agentien, Okklusivmittel, opazifizierende Mittel, oxidierende Mittel, Pestizide, pH-Einstellungsmittel, Plastifizierungsmittel, Konservierungsmittel, Treibmittel, Reduktionsmittel, Hautbleichungsmittel, Hautkonditionierungsmittel, Hautschutzmittel, Abgleitmodifizierungsmittel, Lösungsmittel, Sonnenschutzmittel, Oberflächenmodifizierer, oberflächenaktive Mittel einschließend Reinigungsmittel, emulgierende Mittel, Schaumbooster, Hydrotope, löslich machende Mittel, nichtoberflächenaktive Suspensionsmittel, Absorber für ultraviolettes Licht, Viskositätssteuerungsmittel, Viskositätsabsenkungsmittel, wässrige Viskositätserhöhungsmittel, nicht wässrige Viskositätserhöhungsmittel und Mischungen derselben umfasst.

**11.** Verwendung der transdermalen Vorrichtung nach Anspruch 1 in der Herstellung einer medizinischen Vorrichtung für die Bereitstellung eines schmerzlindernden Effekts für einen bedürftigen Patienten.

**12.** Verwendung nach Anspruch 11, wobei der analgetische Effekt lokal ist.

**13.** Verwendung nach Anspruch 11, wobei der analgetische Effekt systemisch ist.

**14.** Verwendung nach Anspruch 11, wobei eine Gabe der transdermalen Vorrichtung mit Iontophorese einhergeht.

**15.** Verwendung nach Anspruch 11, wobei eine Gabe der transdermalen Vorrichtung mit Magnetophorese einhergeht.

**16.** Verwendung nach Anspruch 11, wobei eine Gabe der transdermalen Vorrichtung mit Sonophorese einhergeht.

**17.** Verwendung nach Anspruch 11, wobei eine transdermale Verabreichung bewirkt wird durch Verwendung einer Lotion, einer Creme oder einer Salbe anstelle einer Vorrichtung.

**18.** Verwendung nach Anspruch 11, wobei eine transdermale Verabreichung bewirkt wird unter Verwendung eines Matrixpflasters, in welchem der Arzneistoff in einem geeigneten druckempfindlichen Haftstoff aufgelöst ist.

**19.** Verwendung nach Anspruch 11, wobei eine Gabe der transdermalen Vorrichtung mit thermischer Ablation einhergeht.

**20.** Verwendung nach Anspruch 11, wobei eine Gabe der transdermalen Vorrichtung mit einer natürlichen Hauttransportvorrichtung von phosphoryliertem Vitamin E einhergeht.

**21.** Verwendung nach Anspruch 11, wobei eine Gabe der transdermalen Vorrichtung mit einer Verwendung von Mikronadeln einhergeht.

## Revendications

**1.** Dispositif transdermique destiné à l’administration transdermique d’une quantité efficace de meptazinol pour procurer

un soulagement antalgique à un mammifère ou à un patient en ayant besoin qui comprend :

(i) une couche de fond ;
(ii) une couche ou un compartiment réservoir ;
(iii) une membrane de régulation ou une membrane microporeuse de non-régulation ; et
(iv) une pellicule adhésive qui est facultativement appliquée sous forme d'anneau périmétrique ou de motif géométrique ou une combinaison de ceux-ci ;
(v) une protection détachable ; et

dans lequel la couche réservoir contient une composition comprenant

(a) une forme de sel de meptazinol selon une quantité qui résulte en la délivrance d'une quantité efficace de meptazinol lorsqu'il est ajouté à un dispositif et que ledit dispositif est appliqué à la peau ; et
(b) un vecteur pharmaceutiquement efficace

**2.** Dispositif transdermique de la revendication 1, dans lequel le dispositif comprend une membrane de régulation et un adhésif et facultativement une protection détachable.

**3.** Dispositif transdermique de la revendication 1, dans lequel la forme de sel est chlorhydrate, hydrobromure, sulfate, phosphate, trifluoroacétate, maléate, tartrate, sulfonate de méthane, sulfonate d'éthane, sulfonate de benzène, sulfonate de P-toluène, sulfonate de naphtalène, sulfonate de camphre, alaninate, asparginate, glutamate et des mélanges de ceux-ci.

**4.** Dispositif transdermique de la revendication 3, dans lequel la forme de sel est le chlorhydrate, le sulfonate de camphre, le sulfonate de toluène, le trifluoroacétate, le maléate ou des mélanges de ceux-ci.

**5.** Dispositif transdermique de la revendication 4, dans lequel le sel de meptazinol possède une solubilité issue du groupe constitué par 30 mg/mL à 500 mg/mL ; 50 mg/mL à 400 mg/mL ; et 75 mg/mL à 300 mg/mL.

**6.** Dispositif transdermique de la revendication 4, dans lequel le sel de meptazinol possède un flux cutané issu du groupe constitué par 20 à 1000 $\mu g/cm^2/h$ ; 50 à 500 $\mu g/cm^2$ ; 125 à 250 $\mu g/cm^2/h$ et 160 à 200 $\mu g/cm^2/h$.

**7.** Dispositif transdermique de la revendication 4, dans lequel le sel de meptazinol est libéré dans un environnement cutané avec un pH issu du groupe constitué par un pH de 2 à 4,0 ; un pH de 4,0 à 7,0, un pH de 4,0 à 6,0 et un pH de 4,0 à 5,0.

**8.** Dispositif transdermique de la revendication 1, qui contient un sel de meptazinol dans lequel la forme de sel est un chlorhydrate, un trifluoroacétate ou des mélanges de ceux-ci, possède une solubilité de 75 mg/mL à 300 mg/mL et un flux cutané de 75 à 250 $\mu g/cm^2/h$.

**9.** Dispositif transdermique de la revendication 6, contenant en outre des abrasifs, des absorbants, des adhésifs, des agents anti-acné, des agents antimottants, des agents anticaries, des agents anti-pelliculaires, des agents anti-moussants, des agents anti-fongiques, des agents anti-microbiens, des anti-oxydants, des agents anti-transpirants, des agents anti-statiques, des liants, des agents tampons, des agents gonflants, des agents chélateurs, des colorants, des éliminateurs de cors/durillons/verrues, des inhibiteurs de corrosion, des astringents cosmétiques, des biocides cosmétiques, des dénaturants, des agents dépilatoires, des astringents médicamenteux, des émollients, des stabilisateurs d'émulsions, des agents dépilatoires, des exfoliants, des antalgiques externes, des agents de formation de pellicules, des agents aromatisants, des ingrédients parfumants, des humectants, des agents lytiques, des agents opacifiants occlusifs, des agents oxydants, des pesticides, des ajusteurs de pH, des plastifiants, des agents conservateurs, des propulsifs, des agents de réduction, des agents d'éclaircissement de la peau, des agents de revitalisation de la peau, des agents de protection de la peau, des modificateurs de glissement, des solvants, des agents de protection solaire, des modificateurs de surface, des surfactants y compris des agents nettoyants, des agents émulsifiants, des accélérateurs de mousse, des hydrotopes, des agents solubilisants, des agents de suspension, des agents de suspension non surfactants, des absorbants de lumière ultraviolette, des agents de contrôle de la viscosité, des agents de diminution de la viscosité, des agents d'augmentation de la viscosité aqueux, des agents d'augmentation de la viscosité non aqueux et des mélanges de ceux-ci.

**10.** Dispositif transdermique de la revendication 8, contenant en outre des abrasifs, des absorbants, des adhésifs, des

agents anti-acné, des agents antimottants, des agents anticaries, des agents anti-pelliculaires, des agents anti-moussants, des agents anti-fongiques, des agents anti-microbiens, des anti-oxydants, des agents anti-transpirants, des agents anti-statiques, des liants, des agents tampons, des agents gonflants, des agents chélateurs, des colorants, des éliminateurs de cors/durillons/verrues, des inhibiteurs de corrosion, des astringents cosmétiques, des biocides cosmétiques, des dénaturants, des agents dépilatoires, des astringents médicamenteux, des émollients, des stabilisateurs d'émulsions, des agents dépilatoires, des exfoliants, des antalgiques externes, des agents de formation de pellicules, des agents aromatisants, des ingrédients parfumants, des humectants, des agents lytiques, des agents opacifiants occlusifs, des agents oxydants, des pesticides, des ajusteurs de pH, des plastifiants, des agents conservateurs, des propulsifs, des agents de réduction, des agents d'éclaircissement de la peau, des agents de revitalisation de la peau, des agents de protection de la peau, des modificateurs de glissement, des solvants, des agents de protection solaire, des modificateurs de surface, des surfactants, y compris des agents nettoyants, des agents émulsifiants, des accélérateurs de mousse, des hydrotopes, des agents solubilisants, des agents de suspension, des agents de suspension non surfactants, des absorbeurs de lumière ultraviolette, des agents de contrôle de la viscosité, des agents de diminution de la viscosité, des agents d'augmentation de la viscosité aqueux, des agents d'augmentation de la viscosité non aqueux et des mélanges de ceux-ci.

**11.** Utilisation d'un dispositif transdermique de la revendication 1 dans la fabrication d'un dispositif médical pour procurer un effet antalgique à un patient en ayant besoin.

**12.** Utilisation de la revendication 11, dans laquelle l'effet antalgique est localisé.

**13.** Utilisation de la revendication 11, dans laquelle l'effet antalgique est systémique.

**14.** Utilisation de la revendication 11, dans laquelle l'administration du dispositif transdermique est accompagnée d'une iontophorèse.

**15.** Utilisation de la revendication 11, dans laquelle l'administration du dispositif transdermique est accompagnée d'une magnétophorèse.

**16.** Utilisation de la revendication 11, dans laquelle l'administration du dispositif transdermique est accompagnée d'une sonophorèse.

**17.** Utilisation de la revendication 11, dans laquelle l'administration transdermique est affectée par l'usage d'une lotion, d'une crème ou d'une pommade à la place d'un dispositif.

**18.** Utilisation de la revendication 11, dans laquelle l'administration transdermique est réalisée en utilisant un timbre à matrice dans lequel le médicament est dissous dans un adhésif approprié sensible à la pression.

**19.** Utilisation de la revendication 11, dans lequel l'administration du dispositif transdermique est accompagnée d'une ablation thermique.

**20.** Utilisation de la revendication 11, dans lequel l'administration du dispositif transdermique est accompagnée d'un transporteur cutané naturel de vitamine E phosphorylée.

**21.** Utilisation de la revendication 11, dans laquelle l'administration du dispositif transdermique est accompagnée de l'utilisation de micro-aiguilles.

**Figure 1 : Comparative permeation of the free base and some selected salts forms of meptazinol through human skin**

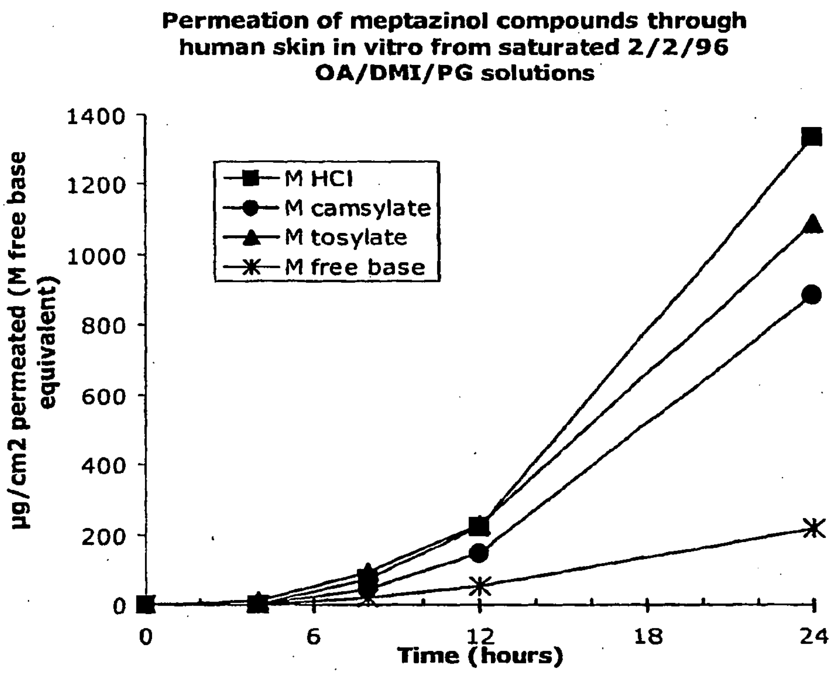


NB Vehicle comprised 2% oleic acid: 2% dimethyl isosorbide: 96% propylene glycol

M HCl = meptazinol hydrochloride

M camsylate = meptazinol camphor sulphonate

M tosylate = meptazinol toluene sulphonate

M free base = meptazinol free base

**Figure 2: Influence of salt form on the permeation of meptazinol through human skin**

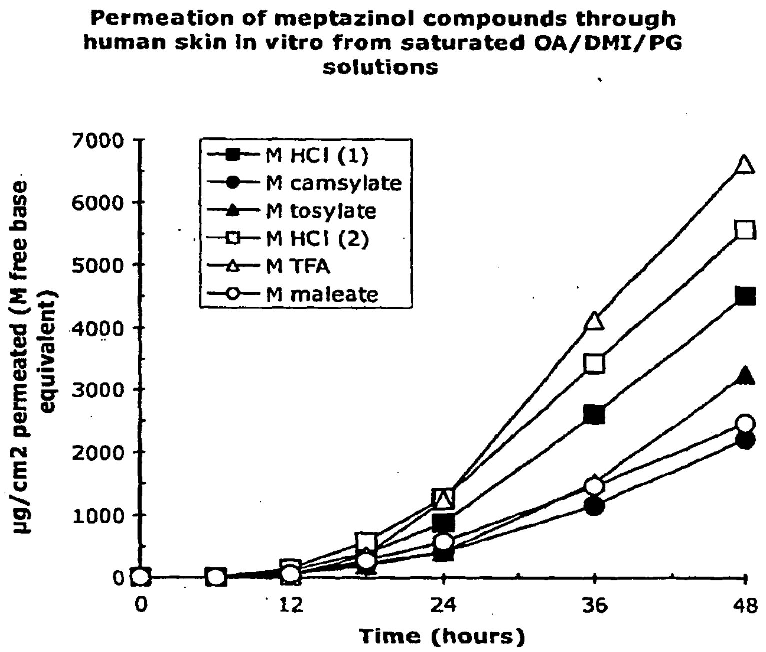


NB Vehicle comprised 2% oleic acid: 2% dimethyl isosorbide: 96% propylene glycol

M HCl (1&2 ) = meptazinol hydrochloride

M camsylate = meptazinol camphor sulphonate

M tosylate = meptazinol toluene sulphonate

M TFA = meptazinol trifluoracetate

M maleate = meptazinol maleate

**Figure 3: Example of meptazinol containing transdermal patch**

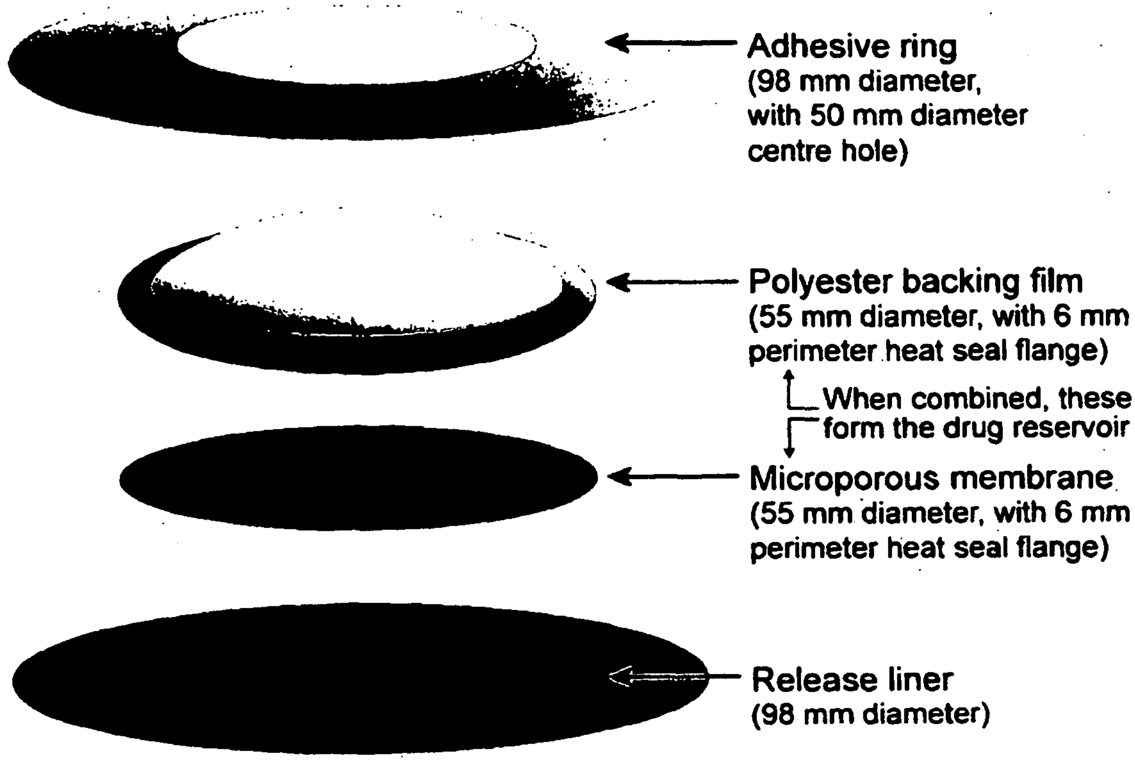

**Figure 4 - Mean meptazinol plasma concentrations (av. 22.73%) on Day 5 in minipigs receiving daily transdermal patch applications containing meptazinol**

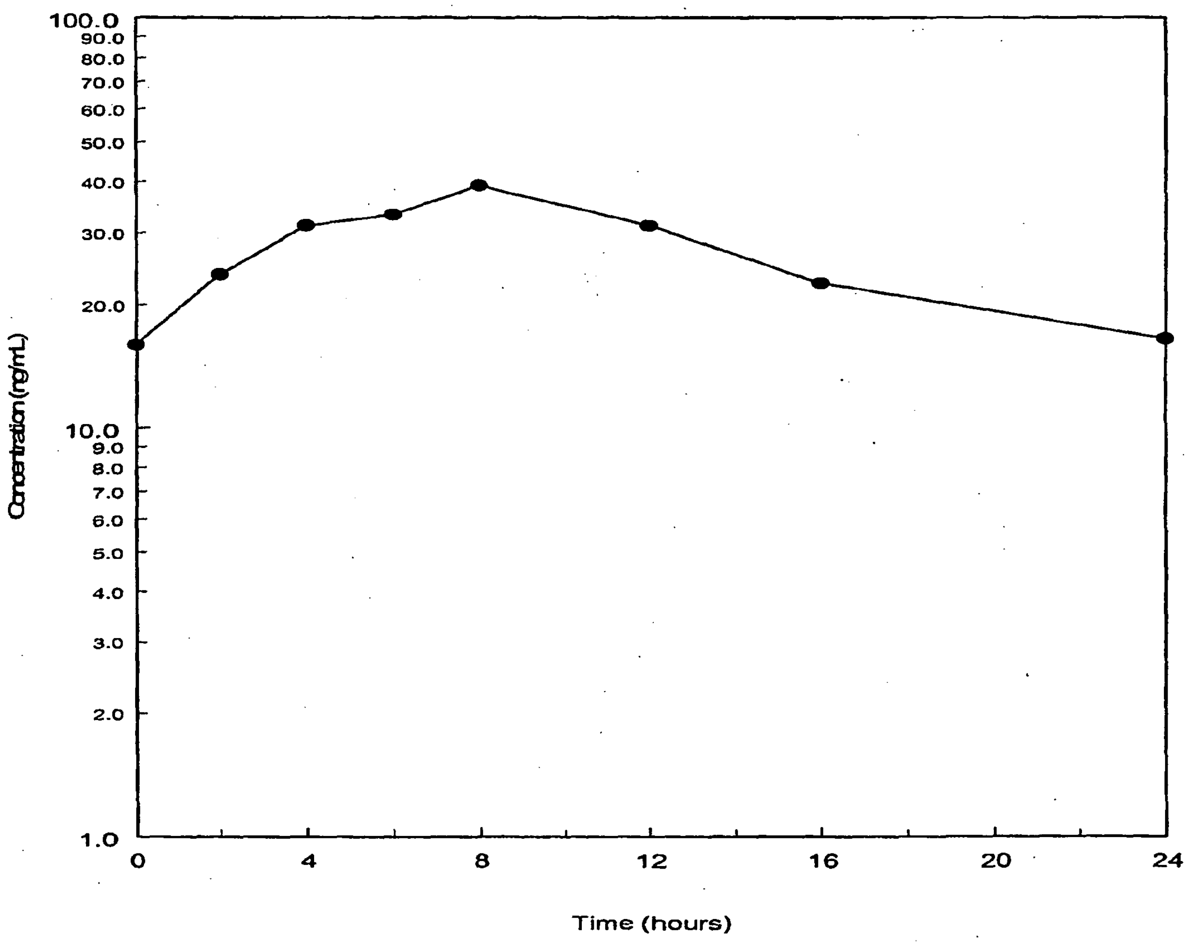

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3729465 A
- US 4197241 A
- GB 2122895 A
- US 6716449 B, Oshlack
- US 20040024006 A, Simon
- US 20040028625 A, Klose
- US 20060029654 A, Cassell
- US 2004033253 A, Shevchuk
- US 2006240128 A, Schlagheck
- US 6818226 B
- US 6791003 B
- US 6787149 B
- US 5858393 A
- US 5612382 A
- US 5464387 A
- US 5023085 A
- US 4891377 A
- US 4654209 A


### Non-patent literature cited in the description


- **Norbury H.M ; Franklin, R.A ; Graham, D.F.** *Eur. J. Clin. Pharm.,* 1983, vol. 25, 77-80
- **Henry et al.** Microfabricated Microneedles: A Novel Approach to Transdermal Drug Delivery. *J. Pharm. Sci.,* 1998, vol. 87, 922-925
- Pharmaceutical Dosage Forms and Drug Delivery Systems. Transdermal Drug Delivery Systems, Ointments, Creams, Lotions and Other Preparations. Williams & Wilkins, 1995, 360
- **Murthy et al.** Physical and Chemical Permeation Enhancers in Transdermal Delivery of Terbutaline Sulphate. *AAPS PharmSciTech.,* 2001, vol. 2 (1
- Pharmaceutical Skin Penetration Enhancement. Marcel Dekker, Inc, 1993
- Penetration Enhancers. **Williams et al.** Adv. Drugs Deliv. Rev. 2004, vol. 56, 603-618
- The International Cosmetic Ingredient Dictionary and Handbook. 2002
- Remington - The Science and Practice of Pharmacy. 2005
- Goodman & Gilman's The Pharmacological Basis of Therapeutics. 2005
- Ansel's Parmaceutical Dosage Forms and Drug Delivery Systems. Lippincott Williams & Wilkins, 2005
- **Roy SD ; Hou SY ; Witham SL ; Flynn GL.** Transdermal delivery of narcotic analgesics: comparative metabolism and permeability of human cadaver skin and hairless mouse skin. *J Pharm Sci.,* 1994, vol. 83 (12), 1723-8